# EUROPEAN PATENT APPLICATION

(11) **EP 2 366 676 A1**
(43) Date of publication of application: **21.09.2011**
(21) Application number: 09824453.6
(22) Date of filing: 06.11.2009
(51) Int. Cl.: C04B 35/486, A61K 6/06

(54) **COLOURED ZIRCONIA MATERIAL, PROCEDURE FOR OBTAINMENT AND APPLICATIONS THEREOF**

(30) Priority: 10.11.2008 ES 200803196
(71) Applicant: Consejo Superior de Investigaciones Cientificas (CSIC), 28006 Madrid (ES)
(72) Inventor: CANAL MENÉNDEZ, María Isabel, E-33011 Oviedo (ES); FERNÁNDEZ VALDÉS, Adolfo, E-33011 Oviedo (ES); TORRECILLAS SAN MILLÁN, Ramón, E-33011 Oviedo (ES)
(74) Representative: Pons Ariño, Angel
(86) International application number: PCT/ES2009/070486
(87) International publication number: WO 2010/052359

(57) **Abstract**

Procedure for obtainment of coloured zirconia, stabilised with yttria, utilising an iron compound as the sole colourant precursor. Furthermore the invention relates to the coloured zirconia material obtainable through said procedure, and use thereof to manufacture dental components, it having a colour similar to that of natural teeth, as catalysts, fuel cell components or structural technical ceramics.

## Description

### FIELD OF THE INVENTION

The present invention relates to a process for obtaining coloured yttria-stabilised zirconia having a colour similar to that of natural teeth. Additionally, the invention relates to coloured zirconia material obtainable by means of said process and use thereof to manufacture dental components, catalysts, fuel cell components or structural technical ceramics.

### STATE OF THE ART

Yttria-stabilised zirconia (Y-TZP) is used in structural and biomedical applications [I. Denry, J.R. Kelly, Dental Materials, 24 (3), 289-298 (2008)] thanks to the reinforcement mechanism of the material and its high flex resistance, its relatively low elastic module and high degree of hardness. The excellent mechanical properties of zirconia and its good chemical stability and biocompatibility [L. Sedel et al., Ann NY Acad Sci., 523 234-256 (1988)] have promoted its use in many biomedical applications, among which that of dental prostheses is one of the most recent, either for crowns or bridges [I. Denry, J.R. Kelly, Dental Materials, 24 (3), 299-307 (2008)].

In the field of dental restorations, the two basic requirements that must be fulfilled by the materials are good aesthetic appearance and high mechanical resistance and, although the mechanical properties of zirconia are the best among all the ceramic materials with possible uses in dental applications, the poor aesthetics thereof (white colour and opaque material) has limited their use to pillars and implants in the past [J. Marzouk, Quintessence Internacional, 27, 533-547 (1995)]. Therefore, from an aesthetic viewpoint, the objective is for the implanted dental crown to be imperceptible to the human eye and, to this end, the material must have a colour similar to that of natural teeth and must be adaptable to each individual tooth or specific positions (molars, frontal teeth, etc.).

Any method developed with the objective of colouring zirconia for use in the dental field must be versatile, in order to provide a sufficiently broad range of colours to satisfy the needs of each individual patient. According to [P.W. Smith, N.H. Wilson, Int J Prosthodont., 11, 302-6. (1998)], the most regularly found colours among patients correspond to the so-called A2 and A3, in accordance with the VITA LUMIN guide used by prosthetic professionals.

There are several methods for improving this aesthetic appearance, including acrylic resin or porcelain coating processes [J.R. Nelly, Annu Rev. Mater Sci., 27, 443-468 (1997)], which simulate the appearance of teeth, providing a colour similar to that of natural teeth, but have the drawback of having low fracture resistance. Additionally, in the case of porcelain coatings, where aesthetic considerations are particularly important, the process must be repeated up to three times: once for the opaque layer, another for the dentin layer and finally for the enamel layer. This entails an important increase in both process time and cost, as well as the risk of fracturing the coating.

Other dental restoration colouring techniques known to exist in patent literature consist of immersing the unit, once pre-sintered and machined, in solutions of metal salt elements. For example, patent US6709694 discloses the use of erbium, praseodymium, iron, copper, nickel or a combination thereof as a colouring solution. These types of techniques allow obtainment of dental units with variable colour intensity and translucence depending on the concentration of salts used. The main problem with this technique is that the dental prosthetist must dedicate time and expense to the colouring process, scrupulously observing the concentration of salts and indicated process times, in order to achieve the expected aesthetic result. Additionally, these types of methods have a considerable disadvantage due to their lack of superficial homogeneity, particularly in peripheral regions, as over-saturation with the colouring pigments tends to occur. An alternative for eliminating this problem of lack of homogeneity is included in US patent application US2007062410, which proposes the addition of a monosaccharide or disaccharide to control the viscosity of the colour solution and therefore, depending on the porosity of the body, work with a viscosity that will ensure total and homogeneous colouring, which would imply an initial study of the porosity of the body and, therefore, a painstaking, long and expensive process.

Alternatively, coloured zirconia can be obtained using powder whereto small quantities of other metallic ions have been added, either in powder form or precursor solution. One example of the possibilities of adding metal ions through a mixture of powders is disclosed in [B. Cales. Bioceramics, 11 (1998)]. In the process followed by the author, the 3% mol yttria-stabilised zirconia is mixed with different colouring additives, both oxides and nitrates, said mixture being subsequently dispersed in alcohol and subjected to a milling process in a ball mill, shaped and sintered in an air atmosphere at 1,500ºC for three hours. However, none of the compositions obtained through this process achieve an ivory colour and the lack of translucence makes it necessary to use a varnish coating, which causes a decrease in flex resistance of the material as well as lengthening the process and raising its cost.

There are a much larger number of literary quotes which make reference to the addition of colourants in the form of dissolved precursors to the zirconia powder to simulate the colour of natural teeth. In this manner, patent applications JP4280864, US5263858 and US6713421 disclose a set of additives to achieve the final effect. In general, these methods require several stages, which implies a lengthier process, greater difficulty in controlling the final colour and greater expense.

A more simplified process would be that disclosed in patent application US2007292597. In this case, the preparation of zirconia powder coated with a colouring agent is mentioned. To this end, a Pr, Fe, Tb and Mn ion solution in a fluidised bed of zirconia powder is added in spray form.

One of the most significant drawbacks in the preparation of coloured zirconias is the impossibility of predicting the final colour of the product. This difficulty is accentuated when the number of added metal ions is increased, as occurs in the aforementioned patent application US2007292597. Even when the colouring agent only contains one type of metal ion, the colour stimulus may not follow a linear relationship with the concentration of colourant added, as in the case of the process disclosed in international patent application WO2007053084. In this case, obtainment of coloured zirconia by adding a single element, praseodymium, is possible. However, the colouring provided by praseodymium is not directly proportional to the quantity of element introduced, which makes it impossible to predict the final colour of the material and, therefore, the necessary shade control is not achieved for developing dental units that suit the individual needs of each patient.

Therefore, the main difficulties in the preparation of coloured zirconias, for use in dental applications, can be summarised in the possibility of achieving an even colour which can be controlled in order to adapt it to each individual patient, and in that the preparation method must be as simple and inexpensive as possible to facilitate scaling to industry level.

### DESCRIPTION OF THE INVENTION

The process of the present invention solves the aforementioned problems achieving, by means of a simple and inexpensive process, control over the final colour of the zirconia, thereby obtaining stabilised zirconia materials, for example, yttria-stabilised zirconia (Y-TZP), which imitate the colour of natural teeth destined for dental applications (crowns, caps, bridges, implants, brackets, etc.) or other applications wherein the colour of the zirconia materials may be an added value.

The doping technique used in the present invention allows sintering of coloured zirconia with functional characteristics identical to those of commercial white zirconia (high density, same mechanical properties and ageing behaviour). Said doping technique implies incorporating a single element as a colourant which, after conventional processing, confers colour thereto, being directly proportional to the quantity of dope introduced.

The purpose of the present invention consists of a doping procedure and process for obtaining coloured zirconia materials, imitating the colour of natural teeth and having the same mechanical properties, ageing resistance and biocompatibility as commercial white zirconias. Additionally, the zirconia obtained using the present process is compliant with ISO 13356:2008 and ISO 6872-1999 standards.

The feasibility of using an atomizer which allows the recovery of gases and solvents has also been verified, being particularly advantageous when working with high-purity alcohols

The process of the present invention enables obtainment of zirconia materials with the desired colour in an inexpensive and accurate manner, preferably colours A2 and A3 of the VITA LUMIN guide used by prosthetic professionals.

Therefore, a first aspect of the present invention relates to a process for obtaining coloured zirconia (hereinafter, process of the invention), which comprises the following stages:
a. preparation of 1,5-5% mol yttria-stabilised zirconia powder;
b. addition of a solution containing an iron compound as the sole colourant precursor to the suspension of step (a), stirring continuously;
c. drying of the suspension resulting from step (b) through an atomisation process;
d. shaping of the product obtained in step (c); and
e. pre-sintering of the product shaped in (d) in an air atmosphere.
   In a preferred embodiment, the process of the invention also comprises the following stage:
f. sintering of the pre-sintered product obtained in step (e) in an air atmosphere.

A preferred embodiment, of the process of the present invention, comprises 2,5-3,5% mol yttria-stabilised zirconia powder; more preferably, 3% mol yttria-stabilised zirconia powder.

In another preferred embodiment of the process of the invention, the liquid medium of the suspension of step (a) of the zirconia powder can be water or alcohol. More preferably, the suspension of step (a) has a solid content ranging between 30% and 70% by weight of the total solution.

Depending on the type of liquid medium, water or alcohol, used in the stabilised zirconia suspension, the iron compound may be inorganic or organic, respectively. Preferably, the iron compound is selected from the list that comprises an alkoxide, chloride, nitrate, oxalate or citrate. Additionally, the iron compound used may be in any state of oxidation; preferably, they may be Fe (II) or Fe (III) compounds, more preferably Fe (II) compounds.

When the medium of the suspension of step (a) is alcohol, the iron compound is preferably an alkoxide and more preferably iron acetylacetonate.

When the medium of the suspension of step (a) is water, the iron compound is preferably a chloride and more preferably iron (II) chloride tetrahydrate (FeCl₂ · 4H₂O).

In step (a) of the process of the invention, the preparation of the stabilised zirconia suspension may be carried out by means of stirring, preferably in a magnetic stirrer-heater, in order to increase the homogeneity of said suspension. Subsequently, on adding the colourant precursor to the suspension, magnetic stirring is maintained for 15-180 minutes, more preferably for 40-80 minutes and even more preferably for 60 minutes.

In this step (b) of the process of the invention an yttria-stabilised zirconia barbotine is formed and homogeneously mixed with the added iron.

In a preferred embodiment of the invention, the barbotine is dried by an atomising air or nitrogen flow, in accordance with the iron compound used as colourant precursor.

After atomising the barbotine, step (c), the shaping of step (d) is carried out. The main objective of the shaping is to give shape and consistency to the powder mass in order to produce an increase in density and, therefore, improve the mechanical properties. There are two ways of carrying out the shaping: applying pressure or applying pressure and high temperatures. The shaping process that can be used is any of those known to a person skilled in the art, such as for example, but not limited to, filter pressing, axial pressing, biaxial pressing, isostatic pressing, extrusioning, colloidal filtering or tape-casting; preferably, shaping is carried out by means of cold isostatic pressing at pressures ranging between 100 MPa and 400 MPa, more preferably at pressures ranging between 150 MPa and 300 MPa.

In a preferred embodiment of the process of the invention, the pre-sintering stage of step (e) is carried out in a conventional oven and in an air atmosphere, preferably at a temperature ranging between 800ºC and 1,400ºC, more preferably at a temperature ranging between 900ºC and 1,100ºC and even more preferably at 1,000ºC.

After the pre-sintering stage of step (e), the body obtained is sintered in a conventional oven under air atmosphere conditions, preferably at temperatures ranging between 1,200ºC and 1,600ºC, more preferably between 1,300ºC and 1,500ºC and even more preferably at 1,400ºC.

A preferred embodiment of the sintering stage of the process of the invention comprises a duration of said stage of more than 10 minutes, more preferably durations of 10-180 minutes and, even more preferably, 60 minutes. In this step, a heating speed of 5-10ºC/min can be used, preferably 5ºC/min.

By means of the process of the invention, a zirconia material with the desired colour is obtained as required by its application, having high density and good mechanical properties.

Therefore, a second aspect of the present invention relates to a coloured zirconia material obtainable by the process of the invention.

In the present invention, "coloured zirconia material" is understood to be the material obtained both after pre-sintering and during sintering. The material obtained after pre-sintering is called blank. These blanks (or pre-sintered disks) are materials which, in turn, may be used before sintering to manufacture the dental components, on being more easily manageable than directly sintered materials.

This coloured zirconia material is characterised in that it has a final density of 98% of the theoretical density of zirconia, a hardness greater than 12 GPa, a flex resistance greater than 800 MPa, a monoclinic phase content of less than 5% after accelerated ageing at 134ºC and a pressure of 2 bar for five hours and a colour wherein the b* colorimetric coordinate is in the range of 0 to 70.

A preferred embodiment of the coloured zirconia material comprises a proportion of iron between 0,001% and 0,5% by weight of the final zirconia material.

Another aspect of the present invention relates to the use of the coloured zirconia material of the invention to manufacture dental components. The dental components may be, but not limited to, implants, crowns, caps or bridges.

A further aspect of the present invention relates to the use of the coloured zirconia material of the invention to manufacture structural technical ceramic elements.

Another aspect of the present invention relates to the use of the coloured zirconia material in the preparation of catalysts or fuel cell components.

Throughout the description and claims, the term "comprises" and its variants do not aim to exclude other technical characteristics, additives, components or steps. For persons skilled in the art, other objects, advantages and characteristics of the invention will be partially inferred from the description and partially inferred from the practical implementation of the invention. The following examples and figure are provided by way of illustration and are not intended to be limiting of the present invention.

### DESCRIPTION OF THE FIGURE

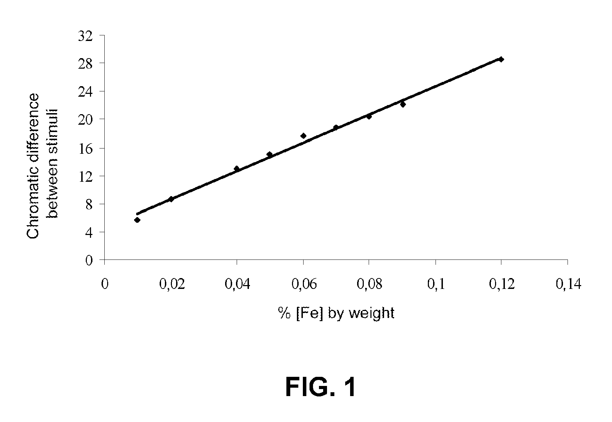

Figure 1 shows the linear relationship between the concentration of iron introduced during doping and the b* colorimetric coordinate (CIELAB system). The linear relationship is represented by the linear correlation coefficient R²= 0,9939.

### EXAMPLES OF EMBODIMENT

The invention is illustrated below by means of trials conducted by the inventors, which demonstrate the effectiveness of the process of the invention for obtaining an iron-doped zirconia material having a controllable colour.

### EXAMPLE 1

The initial raw materials used were:
- Oxidic powder: Tosoh Zirconia (tetragonal zirconia, TZ-3Y-E, with an average particle size of 260Å and 5,6% by weight of Y₂O₃).
- Iron (II) chloride tetrahydrate (>98%).
- Distilled water.

100 g of zirconia were used, dispersed in 60 g of water. Subsequently, a solution of 0,231 g of iron chloride tetrahydrate in 50 g of water was added drop by drop and stirred for 60 minutes.

The barbotine thus prepared was dried by means of an atomised air flow, obtaining granulated zirconia which included the colouring agent distributed in a homogeneous manner.

The dry product thus obtained was subjected to a shaping process by means of cold isostatic pressing at 200 MPa, obtaining a green body which was pre-sintered in a conventional oven and air atmosphere at a temperature of 1,000ºC.

Finally, the zirconia unit was sintered in a conventional oven at 1,400ºC for 60 minutes and a heating ramp rate of 5ºC/min.

The resulting material was characterised by its density, grain size, hardness, fracture resistance, ageing and colour (Table I). Density was determined using the Archimedes method, giving a value of 99,08% with respect to the theoretical density of zirconia, which was 6,1 g/cc. The grain size of the doped zirconia was 390 nm. The material was subjected to a hardness test using a Buehler Micromet 5103 microdurometer, giving a value of 13,6 GPa. Its fracture resistance was determined by means of a four-point flex test using INSTRON 8562 equipment, giving a value of 997 MPa. Ageing resistance was determined using a TUTTNAUER 2540EL autoclave, after treatments at 134ºC and 2 bars of pressure, and a monoclinic fraction percentage of less than 5% was found after five hours of treatment, the limit established in the ISO 13356:2008 standard being a monoclinic fraction percentage equal to or less than 25% after five hours of accelerated ageing. The colour was characterised by a MINOLTA CR200 colorimeter and the parameters recorded in accordance with the CIELAB colour model were L=79,80 a*=0,10 b*=19,22.

**Table I.- Results obtained after characterisation of the zirconia doped with 650ppm of Fe and comparison thereof with undoped zirconia.**

| **Property** | | **Zirconia doped with 0,065% by weight of Fe** | **Undoped zirconia** |
|---|---|---|---|
| Density % | | 99,08 | 99,10 |
| Grain size (nm) | | 390 | 380 |
| Hardness (Gpa) | | 13,60 | 13,59 |
| Fracture resistance (Mpa) | | 997 | 1052 |
| Ageing monoclinic fraction % after 5 hours of treatment in autoclave (134ºC, 2 bar) | | 4,35 | 2,15 |
| Colour | L | 79,80 | 93,54 |
| | a* | -0,10 | -1,09 |
| | b* | 19,22 | 1,62 |

As can be observed, this coloured zirconia material is characterised in that it has mechanical properties and ageing resistance similar to that of uncoloured Y-TZP materials.

### EXAMPLE 2

Through the process of the invention it was possible to obtain, in an inexpensive and accurate manner, zirconia materials with the desired colour, for example colours A2 and A3 of the VITA LUMIN guide used by prosthetic professionals.

Therefore, different materials were manufactured (see Figure 1, Table II and Table III), modifying the initial quantity of Fe, while the conditions were the same as those described in Example 1).

**Table II.- List of colours obtained by means of doping and required concentration of iron.**

| **[Fe] % by weight** | **L** | **a*** | **b*** |
|---|---|---|---|
| 0 | 93,54 | -1,09 | 1,62 |
| 0,025 | 83,90 | -1,00 | 13,40 |
| 0,065 | 79,80 | -0,10 | 19,22 |
| 0,12 | 77,33 | 1,39 | 21,75 |

**Table II.- List of colours obtained by means of doping and those corresponding to the VITA LUMIN guide used by prosthetic professionals.**

| **Doped zirconia** | | **Vita Lumin Guide** | |
|---|---|---|---|
| 0,025% by weight of Fe | L=83,9 | A1 | L=82,4 |
| | a*=-1,0 | | a*=-1,4 |
| | b*=13,40 | | b*=14,3 |
| 0,065% by weight of Fe | L=79,8 | A2 | L=79,1 |
| | a*=-0,1 | | a*=0,6 |
| | b*=19,22 | | b*=19,2 |
| 0,120% by weight of Fe | L=77,33 | A3 | L=77,6 |
| | a*=1,39 | | a*=1,0 |
| | b*=21,75 | | b*=21,00 |

### EXAMPLE 3

The initial raw materials used were:
- Oxidic powder: Tosoh Zirconia (tetragonal zirconia, TZ-3Y-E, with an average particle size of 260Å and 5,6% by weight of Y₂O₃).
- Iron (II) acetylacetonate (99,95%).
- Anhydrous ethanol (99,97%).

100 g of zirconia were used, dispersed in 60 g of ethanol. Subsequently, a solution of 0,114 g of iron (II) acetylacetonate in 50 g of anhydrous ethanol was added drop by drop and stirred for 60 minutes.

The barbotine thus prepared was dried by means of an atomised nitrogen flow, obtaining granulated zirconia which included the colouring agent distributed in a homogeneous manner, while recovering the solution.

The dry product thus obtained was subjected to a shaping process by means of cold isostatic pressing at 200 MPa, obtaining a green body which was pre-sintered in a conventional oven and air atmosphere at a temperature of 1,000ºC.

Finally, the zirconia unit was sintered in a conventional oven at 1,400ºC for 60 minutes and a heating ramp rate of 5°C/min.

The resulting material was characterised by its density, grain size, hardness, fracture resistance, ageing and colour in accordance with the methods detailed in Example 1. Density was 99,21% with respect to the theoretical density of zirconia. The grain size of the doped zirconia was 350 nm. Hardness was 13,8 GPa and fracture resistance was 965 MPa. After five hours of accelerated ageing at 134ºC and a pressure of 2 bar, a monoclinic fraction percentage of less than 5% was found (the quantity of the monoclinic phase established in the ISO 13356:2008 being less than or equal to 25% after accelerated ageing for five hours). The parameters recorded in accordance with the CIELAB colour model were L=83,9 a*=-1,00 b*=13,40.

## Claims

1. A process for obtaining coloured zirconia which comprises the following stages:
a. preparation of 1,5-5% mol yttria-stabilised zirconia powder;
b. addition of a solution containing an iron compound as the sole colourant precursor to the suspension of step (a), stirring continuously;
c. drying of the suspension resulting from step (b) through an atomisation process;
d. shaping of the product obtained in step (c); and
e. pre-sintering of the product shaped in (d) in an air atmosphere.

2. A process, according to claim 1, which also comprises the following stage:
f. sintering of the pre-sintered product obtained in step (e) in an air atmosphere.

3. A process, according to any of claims 1 or 2, wherein the zirconia powder is 2,5-3,5% mol yttria-stabilised.

4. A process, according to claim 3, wherein the zirconia powder is 3% mol yttria-stabilised.

5. A process, according to any of claims 1 to 4, wherein the suspension of step (a) is prepared using water.

6. A process, according to claim 5, wherein the iron compound is selected from among a chloride or a nitrate.

7. A process, according to claim 6, wherein the iron compound is iron chloride tetrahydrate.

8. A process, according to any of claims 1 to 4, wherein the suspension of step (a) is prepared using alcohol.

9. A process, according to claim 8, wherein the iron compound is selected from among the list that comprises an alkoxide, oxalate or citrate.

10. A process, according to claim 9, wherein the iron compound is iron acetylacetonate.

11. A process, according to any of claims 1 to 10, wherein the magnetic stirring of step (b) is maintained for 15-180 minutes.

12. A process, according to claim 11, wherein the stirring is maintained for 40-80 minutes.

13. A process, according to any of claims 1 to 12, wherein the solid content of the zirconia suspension of step (a) is in the interval of 30-70% by weight.

14. A process, according to any of claims 1 to 13, wherein atomisation is carried out using an air or nitrogen flow.

15. A process, according to any of claims 1 to 14, wherein the shaping of step (d) is carried out by means of cold isostatic pressing at pressures ranging between 100 M Pa and 400 M Pa.

16. A process, according to any of claims 1 to 15, wherein the pre-sintering stage of step (e) takes place at a temperature of 800-1,400ºC.

17. A process, according to claim 16, wherein the pre-sintering stage takes place at a temperature of 900-1,100ºC.

18. A process, according to any of claims 2 to 17, wherein the material is sintered at temperatures of 1,200-1,600ºC.

19. A process, according to claim 18, wherein sintering takes place at a temperature of 1,300-1,500ºC.

20. A process, according to any of claims 2 to 19, wherein the duration of the sintering stage is longer than 10 minutes.

21. Coloured zirconia material obtainable through the process according to any of claims 1 to 20.

22. Coloured zirconia material, according to claim 21, **characterised in that** it has a final density greater than 98% of the theoretical density of zirconia, a hardness greater than 12 GPa, a flex resistance greater than 800 MPa, a monoclinic phase content of less than 5% and a colour wherein the b* colorimetric coordinate is in the range of 0 to 70.

23. Coloured zirconia material, according to any of claims 21 or 22, wherein the proportion of iron ranges between 0,001-0,5% by weight of the final material.

24. Use of the coloured zirconia material, according to any of claims 21 to 23, to manufacture dental components.

25. Use of the coloured zirconia material, according to claim 24, wherein the dental components are implants, crowns, caps or bridges.

26. Use of the coloured zirconia material, according to any of claims 21 to 23, to manufacture structural technical ceramic elements.

27. Use of the coloured zirconia material, according to any of claims 21 to 23, to prepare catalysts or fuel cell components.
